# EUROPEAN PATENT APPLICATION

(11) **EP 1 118 618 A1**
(43) Date of publication of application: **25.07.2001**
(21) Application number: 99940632.5
(22) Date of filing: 02.09.1999
(51) Int. Cl.: C07K 2/00, C12N 15/10, C12Q 1/02, G01N 33/50

(54) **MONITOR PROTEIN FOR MEASURING PHOSPHORYLATION OF PROTEIN**

(30) Priority: 02.09.1998 JP 24886198
(71) Applicant: Center for Advanced Science and Technology Incubation, Ltd, Tokyo 100-0005 (JP)
(72) Inventor: HAGIWARA, Masatoshi, Medical Research Institute, Bunkyo-ku, Tokyo 113-0034 (JP); INOUYE, Satoshi, Chisso Corporation, Yokohama-shi, Kanagawa 236-8605 (JP); NAGAI, Yasuo, Medical Research Institute, Bunkyo-ku, Tokyo 113-0034 (JP)
(74) Representative: Grünecker, Kinkeldey, Stockmair & Schwanhäusser Anwaltssozietät
(86) International application number: JP9904769
(87) International publication number: WO0014108

(57) **Abstract**

A phosphorylation monitoring protein which can easily monitor the phosphorylation of a phosphorylation region by using a protein comprising the phosphorylation region and a variable property region whose properties change according to the conformational change of the phosphorylation region, has been successfully produced. In the present invention, an analysis system in which no radioactive isotopes are used and that is applicable to *in vivo* measurement has been developed for protein phosphorylation. The analysis system of the present invention can be used not only for the detection of phosphorylation reaction, but also for screening a kinase and screening a compound which stimulates or inhibits phosphorylation.

SEQUENCE LISTING

<110> CENTER FOR ADVANCED SCIENCE AND TECHNOLOGY INCUBATION, LTD.

<120> Monitor proteins for measurement of protein phosphorylation

<130> SEN-001PCT

<150> JP 1998-248861
<151> 1998-09-02

<160> 8

<210> 1
<211> 23
<212> PRT
<213> Artificial Sequence

<220>
<223> Description of Artificial Sequence: CREB
      phosphorylation region.

<400> 1
Thr Ser Ser Glu Ile Leu Ser Arg Arg Pro Ser Tyr Arg Lys Ile Leu
  1               5                  10                  15
Asn Asp Leu Ser Ser Asp Thr
            20

<210> 2
<211> 23
<212> PRT
<213> Artificial Sequence

<220>
<223> Description of Artificial Sequence: Kemptide
      phosphorylation region.

<400> 2
Thr Ser Leu Arg Arg Ala Ser Leu Gly Thr Gly His Ala Val Arg Ala
  1               5                  10                  15
Ile Gly Arg Leu Ser Ser Thr
             20

<210> 3
<211> 73
<212> DNA
<213> Artificial Sequence

<220>
<223> Description of Artificial Sequence: 'LCR-1B',
      a template for generation of CREB phosphorylation region.

<400> 3
ccggtatccg atgacagatc gttcaggatc ttgcgatatg atggacgtcg cgacaggatc 60
tctgatgagg tac                                                    73

<210> 4
<211> 73
<212> DNA
<213> Artificial Sequence

<220>
<223> Description of Artificial Sequence: 'LKe-1B',
      a template for generation of Kemptide
      phosphorylation region.

<400> 4
ccggtggacg acaatcgtcc gatagcgcgt actgcgtgac cggttcctaa cgatgctcga 60
cgcaatgagg tac                                                    73

<210> 5
<211> 23
<212> DNA
<213> Artificial Sequence

<220>
<223> Description of Artificial Sequence: 'PCR-1K',
      a PCR-primer for generation of CREB
      phosphorylation region.

<400> 5
tgctggtacc tcatcagaga tcc                                              23

<210> 6
<211> 23
<212> DNA
<213> Artificial Sequence

<220>
<223> Description of Artificial Sequence: 'PCR-1A',
      a PCR-primer for generation of CREB phosphorylation region.

<400> 6
agcaccggta tccgatgaca gat                                              23

<210> 7
<211> 23
<212> DNA
<213> Artificial Sequence

<220>
<223> Description of Artificial Sequence: 'PKe-1K',
      a PCR-primer for generation of Kemptide
      phosphorylation region.

<400> 7

tgctggtacc tcattgcgtc gag 23

<210> 8
<211> 23
<212> DNA
<213> Artificial Sequence

<220>
<223> Description of Artificial Sequence: 'PKe-1A',
a PCR-primer for generation of Kemptide
phosphorylation region.

<400> 8
atcaccggtg gacgacaatc gtc 23

## Description

### Technical Field

The present invention relates to a protein for measuring an activity of phosphorylating a protein and a nucleic acid encoding the protein.

### Background Art

Ingenious switch mechanisms are present in a living body of an organism, and these switch mechanisms control, for example, differentiation, development, protective response, metabolic activity, etc. As one of these switch mechanisms, a phenomenon called protein phosphorylation is known (Hunter T., 1991, Methods Enzymol. 200: 3-37). Phosphorylation is mediated by an enzyme. Specifically, this enzyme introduces a phosphate group to a substrate protein to activate this substrate protein. This activated substrate protein induces diverse reactions, for instance, regulation of gene expression and cellular proliferation.

Recently, a cascade mechanism called cellular signal transduction, which is a base for diverse reactions in a living body, including cellular development and differentiation, have been uncovered. In this cellular signal transduction pathway, the above phosphorylation reaction has been shown to play an important role as one of the switch mechanisms which determine the on-state and off-state of the pathway (Hunter T., 1995, Cell 80: 225-236).

So far, it has been revealed that multiple pathways are present in signal transduction pathways. For instance, a relatively simple pathway through which an extracellular information directly passes a cell membrane to be transmitted into the inside of a cell, such as a hormone, is one of signal transduction pathways, while recently, complex pathways through which a given information is transmitted into a cell by communication among multiple molecules have been discovered.

One example is a signal transduction pathway mediated by G protein (Gilman, A. G., 1987, Annu. Rev. Biochem. 56: 615-649). Inthis pathway, binding of a specific substance to a receptor formed on a cell membrane is a signal for initiating the transduction pathway. On the other hand, G protein conjugated with the receptor in a cell elevates the intracellular concentration of cyclic-adenosine mono phosphate (cAMP), which is a second messenger. A specific kinase phosphorylates A kinase by using the increased concentration of cAMP. It is proposed that phosphorylation of A kinase generated in this manner, namely an activation, is transmitted to, for example, the nucleus, as a signal, inducing the expression, such as a protein necessary for an *in vivo* response at the final stage of the cascade response. Other than this signal transduction pathway mediated by G protein, for instance, a transduction pathway based on a cell surface receptor and MAP kinase (Madhani, H. D. et al., 1998, Trends Genet. 14: 151-155) and a transduction pathway based on a cell membrane phospholipid and C kinase (Weinstein, I. B. et al., 1997, Adv. Exp. Med. Biol. 400A: 313-321) are known, indicating that protein phosphorylation plays an important role as a switch for activating a pathway in all cases. Many studies have suggested, however, that the signal transduction pathways known at present are only a part of diverse transduction pathways expected to be present in a cell.

Thus, uncovering signal transduction pathways is important in revealing various mechanisms in life activities. Novel transduction pathways, and substances involved in known transduction pathways have been searched and functions of these substances have been analyzed. Among these, analyses on phosphorylation reaction playing an important role as a switch mechanism in signal transduction, search for enzymes carrying on this reaction, and such, have been widely studied.

Enzymes carrying on such protein phosphorylation reaction have been searched mainly by comparing homology with genes encoding known protein kinases. In a kinase family which phosphorylates a tyrosine residue, a consensus sequence in this family is known (Hunter T. et al., 1984, Adv. Cyclic Nucleotide Protein Phosphorylation Res. 17: 443-455), and thus, for example, screening libraries by using a probe designed based on this consensus sequence enables searching a novel enzyme. Whether an enzyme newly discovered in this manner has an activity to phosphorylate a protein is mainly analyzed by *in vitro* analysis system using ³²P isotope (D. Grahame Hardie, Eds. "Protein Phosphorylation: A Practical Approach," 1993, Oxford University Press). Specifically, a searched enzyme, a substrate protein or a known peptide comprising a phosphorylation region, and a ³²P phosphate are mixed to determine whether the ³²P is added to the substrate protein or the peptide by monitoring radioactivity of a reaction product.

Conventional methods for analyzing protein phosphorylation, however, require the use of radioactive isotopes, such as ³²P, limiting experimental facilities and such, requiring a careful experimental manipulation, and thus making the manipulation cumbersome. In addition these kinds of experiments accompany waste of radioactive isotopes, and therefore, are not preferable for the natural environment. Moreover, the above analytical method does not enable directly measuring the intracellular activity, and thus, has a problem of giving only results measured under the artificial environment.

To solve these problems in *in vitro* analysis system, *in vivo* measurement system has been developed. The method does not directly measure phosphorylation, but measures protein phosphorylation reaction by using cellular proliferation induced by phosphorylation as an indicator. More specifically, in this analysis system, a cellular proliferation rate is measured by uptake of ³H thymidine into a cell, and, based on the cellular proliferation rate, phosphorylation reaction at the early stage in signal transduction pathway is measured. In this method, however, rapid measurement of phosphorylation reaction is difficult because the activity at the end of transduction pathways, namely, cellular proliferation is measured as an indicator. Moreover, the method is not suitable for measuring timing of occurrence of phosphorylation reaction and such.

On the other hand, an analysis system by measuring calcium concentration *in vivo* has been recently reported (Miyawaki, A. et al., 1997, Nature 388: 882-887). This system, in which a fusion protein in which green fluorescent proteins (GFP) of luminous jellyfish, *Aequorea* victoria, are fused to both ends of a calmodulin protein is used for measurement, utilizes the characteristic that fluorescence is emitted when GFP proteins at the both ends approach and interact with each other. More specifically, binding of a certain amount of calcium to the central calmodulin protein alters conformation of the calmodulin protein, and the GFP proteins at the both ends interact with each other to emit fluorescence. *In vivo* calcium concentration can be determined by measuring the emitted light. In the measurement of calcium concentration, such a superior method in which radioactive isotopes are not used and that is applicable to the *in vivo* measurement has been developed. However, in the above protein phosphorylation reaction, development of such a system is behind.

### Disclosure of the Invention

An objective of the present invention is to establish an analysis system for protein phosphorylation, the analysis system in which radioactive isotopes are not used and that is applicable to the *in vivo* measurement.

The present inventors first obtained genes encoding CREB phosphorylation sequence (Gonzalez, G. A. et al., 1989, Cell 59: 675-680) and kemptide phosphorylation sequence (Kemp B. E. et al., 1977, J. Biol. Chem. 252: 4888-4894) as representative examples of amino acid sequences known to be phosphorylated, and prepared plasmid "pETIC-ART" and "pETIC-Kempart", respectively, by inserting each gene between RSGFP site (also called RGFP) and BSGFP site (also called BGFP) of a plasmid pETIC (Romoser V. A. et al., 1997, J. Biol. Chem. 272: 13270-13274). Both plasmids can express a fusion protein comprising a structure of "RSGFP - a protein phosphorylation sequence - BSGFP" and are different only in the protein phosphorylation sequences. As a negative control in the phosphorylation reaction experiment, pETIC-1 was used. This plasmid can express a fusion protein comprising the structure composed of "RSGFP - a calmodulin binding site - BSGFP" (Romoser V. A. et al., J. Biol. Chem., 1997, 272: 13270-13274).

*E. coli* BL-21(DE3) was transformed with pETIC-ART, pETIC-Kempart, or pETIC-1, and the above fusion proteins were extracted from the microbial cells after incubation.

Analysis by using *in vitro* analysis system with ³²P isotope found that the fusion proteins derived from pETIC-ART and pETIC-Kempart were phosphorylated but the fusion protein derived from pETIC-1 was not.

Whether fluorescence changed or not was examined in the case that the reaction was carried out using a non-radioactive phosphate as a substrate without ³²P isotope. As a result, depending on the presence or absence of phosphorylation reaction, the fusion protein derived from pETIC-ART showed the differences in absorption wavelength, however, the presence or absence of phosphorylation in the fusion proteins derived from pETIC-Kempart or pETIC-1 did not lead to differences in absorption wavelength. These results uncovered the following two points.
1. Phosphorylation in the fusion protein derived from "pETIC-ART" generates protein conformational change necessary for generating changes in GFP fluorescence.
2. The fusion protein derived from "pETIC-Kempart" is phosphorylated, however, this phosphorylation does not generate protein conformational change necessary for generating changes in GFP fluorescence.

Specifically, the present inventors have shown that, in some cases, protein phosphorylation generates conformational change in a protein. The combination of two kinds of GFP proteins described above is an example for detecting conformational change as a property change, and it is obviously possible that the same system can be constructed for proteins having other properties, etc.

Then, a protein in which a region for monitoring property change, namely a variable property region, and a region to be phosphorylated, namely a phosphorylation region, are fused can be used as a protein for monitoring protein phosphorylation (a monitor protein). This case does not require the use of radioactive isotopes and is applicable to *in vivo* measurement. In fact, the present inventors have successfully monitored phosphorylation in real time in living cells into which a vector expressing a monitor protein has been introduced.

Such a monitor protein can easily monitor protein phosphorylation, thereby can be used for a method for screening a novel kinase and such. Moreover, using this system, a compound which stimulates or inhibits phosphorylation can be screened.

The finding that some protein kinases generate conformational change, and others do not enable determining whether protein phosphorylation generates conformational change or not by using both an existing *in vitro* analysis system with ³²P isotope, and a method using property change of a monitor protein. More specifically, the present invention relates to:
(1) a monitor protein for measuring protein phosphorylation, the monitor protein comprising (a) a phosphorylation region comprising an amino acid residue or an amino acid sequence to be phosphorylated, and (b) a variable property region showing a property change attributed to a conformational change of a protein comprising at least the phosphorylation region, which conformational change is caused by phosphorylation of the amino acid residue or the amino acid sequence;
(2) the monitor protein of (1), wherein the variable property region is a protein that emits fluorescence;
(3) the monitor protein of (1) or (2), wherein the variable property region is bound to each of both ends of the phosphorylation region;
(4) the monitor protein of (3), wherein the variable property region comprises RSGFP and BSGFP which are comprised in green fluorescent protein (GFP) of *Aequorea victoria*;
(5) the monitor protein of any one of (1) to (4), wherein the phosphorylation region comprises the amino acid sequence of SEQ ID NO: 1;
(6) a nucleic acid encoding the monitor protein of any one of (1) to (5);
(7) an expression vector carrying the nucleic acid of (6);
(8) a method for measuring phosphorylation ability in a cell by introducing the monitor protein of any one of (1) to (5), the nucleic acid of (6), or the expression vector of (7) into the cell;
(9) a method for measuring phosphorylation ability of a test protein, the method comprising reacting the test protein with the monitor protein of any one of (1) to (5), and measuring a property change of the monitor protein;
(10) a method for screening a kinase, the method comprising:
   (a) reacting a test protein with the monitor protein of any one of (1) to (5),
   (b) measuring the property change of the monitor protein, and
   (c) selecting the test protein which alters the property of the monitor protein;
(11) a method for screening a compound which stimulates or inhibits phosphorylation, the method comprising:
   (a) contacting, in the presence of a test sample, a kinase with the monitor protein of any one of (1) to (5), the monitor protein comprising a phosphorylation region to be phosphorylated by the kinase,
   (b) measuring the property change of the monitor protein, and
   (c) selecting a compound which stimulates or inhibitsthe property change in comparison with the property change in the absence of the test sample; and
(12) a method for screening a compound which stimulates or inhibits phosphorylation, the method comprising:
   (a) preparing a cell into which the expression vector of (7) is introduced,
   (b) measuring, in the presence of a test sample, the property change of a monitor protein expressed in the cell, and
   (c) selecting a compound which stimulates or inhibits the property change in comparison with the property change in the absence of the test sample.

The present invention is illustrated in detail below.

### 1. A monitor protein for measuring protein phosphorylation

A monitor protein for measuring protein phosphorylation (a monitor protein, hereafter) is a protein comprising a "phosphorylation region" and one or more "variable property regions."

Herein, a "phosphorylation region" means a region comprising an amino acid residue to be phosphorylated and capable of changing its conformation by phosphorylation of the amino acid residue. Among the following proteins known to be phosphorylated, those capable of showing conformational change by phosphorylation can be used as a phosphorylation region. As the protein to be phosphorylated, CREB transcription factor (Hagiwara M. et al., 1993, Mol. Cell. Biol. 13: 4852-4859) and ATF1 (Shimomura, A et al., 1996, J. Biol. Chem. 271: 17957-17960) are exemplified.

For example, a conformational change can be induced even in a protein, which does not usually undergo any conformational change by phosphorylation, by fusing the monitor protein with a phosphate group-specific antibody. Therefore, a phosphate group-specific antibody can be combined with another property variable region.

A partial sequence comprising an amino acid residue to be phosphorylated can be preferably used as well as a full length of the protein comprising the phosphorylation region. For instance, in the case of CREB transcription factor, it is known that serine at 133rd residue is phosphorylated by protein kinase A. Therefore, any partial sequence of CREB transcription factor can be used as long as it contains the 133rd serine residue and is capable of being phosphorylated. For example, as the partial sequence, an amino acid sequence of SEQ ID NO: 1 can be selected. As an "amino acid residue to be phosphorylated," for example, tyrosine, threonine, and so on can be used other than serine.

Any amino acid sequence can be used as a "variable property region" as long as its phosphorylation can be easily determined. An example is GFP.

"Property variable regions" can be separately present as long as forming a fusion protein with a phosphorylation region. Amonitor protein can be preferably constructed as a "measurement protein pair" provided at both ends of a phosphorylation region. More specifically, an amino acid residue in a phosphorylation region is phosphorylated to alter the conformation of the phosphorylation region, and a measurement protein pair prepared at both the ends of the phosphorylation region interacts toshowameasurableproperty. For example, each component can be designed as follows.

Any "measurement protein pair" provided at both ends of the above phosphorylation region can be used as long as showing a measurable property by interacting with each other due to conformational change of the above phosphorylation region. For example, proteins emitting fluorescence by interacting with each other, such as BSGFP and RSGFP of *Aequorea victoria,* can be preferably used.

Any desirable functional regions can be added to a monitor protein other than the above "phosphorylation region" and "variable property region." Examples include a nuclear localization signal to transport a monitor protein to the nucleus for measuring phosphorylation in the nucleus (Goldfarb, D. S. et al., 1986, Nature 322: 641-644) and a marker which is an indicator for the introduction of a monitor protein into a cell (Heim, R. et al., 1995, Nature 373: 663-664).

### 2. Production of a monitor protein

Any methods for producing the above monitor protein can be used. For example, a monitor protein can be produced by expressing a nucleic acid encoding the monitor protein in any host cell, such as *E*. *coli.* More specifically, this "nucleic acid encoding a monitor protein" is constructed so that a measurement protein pair and a phosphorylation region can be expressed as a fusion protein by ligating a nucleotide sequence encoding the above variable property region and a nucleotide sequence encoding the phosphorylation region in the same reading frame. A monitor protein can be amplified and produced by ligating the nucleic acid encoding the monitor protein constructed above to any vector, introducing the vector into an appropriate host cell, and expressing the nuclei acid. A monitor protein produced in this manner can be used for measuring *in vitro* or *in vivo* phosphorylation directly or preferably after isolation and purification.

Any combination of a vector and a host cell can be used for producing a monitor protein, and the nucleic acid encoding a monitor protein can be ligated downstream of a promoter with high expression activity for improving monitor protein expression. Such a promoter and the like can be arbitrary selected from known promoters and used.

### 3. A nucleic acid encoding a monitor protein

The above "nucleic acid encoding a monitor protein" can include those contain another sequence as long as encoding the monitor protein. Another sequence includes a sequence effectively expressing a monitor protein in a cell, for example, a regulatory sequence, such as a promoter and enhancer; a selective gene, such as a drug resistance marker for detecting introduction of the nucleic acid into cells; etc.

This nucleic acid can be used for directly detecting phosphorylation reaction in a cell by introducing the nucleic acid into the cell and directly expressing the above monitor protein as well as for producing the monitor protein. The monitor protein can be transported into a cell more effectively by introducing the nucleic acid encoding the monitor protein into the cell, because, in general, nucleic acids can be introduced into a cell more effectively than proteins. In addition, as described later, the above nucleic acid can be easily transported into a cell and prepared by harboring the nucleic acid in a vector.

### 4. A vector carrying a nucleic acid encoding a monitor protein

Any vector can be used as long as being capable of self-replication and containing a transcription initiation sequence. Any known plasmids which can be expressed in, for example, *E. coli,* yeast, plant cells, insect cells, mammalian cells, and such can be used. Therefore, a plasmid can be appropriately selected from these known plasmids, corresponding to, for example, a cell to be examined.

### 5. A monitor protein for screening a kinase

A monitor protein can be used for searching and screening an enzyme which phosphorylates a phosphorylation region in the monitor protein. An enzyme which phosphorylates a phosphorylation region can be screened by acting a monitor protein comprising the desirable phosphorylation region on a test protein *in vivo* or *in vitro* and detecting phosphorylation of the monitor protein. In the case of screening *in vivo,* a vector carrying a nucleic acid encoding a monitor protein can be used. Any test proteins can be used. Natural protein libraries, artificial protein libraries, cDNA libraries thereof, and such can be used. Alternatively, for example, ribozyme libraries can be used.

### 6. Amonitor protein for screening a compound which stimulates or inhibits phosphorylation

A monitor protein can be used for screening an accelerator and inhibitor of enzyme activity which phosphorylates or dephosphorylates a phosphorylation region in the monitor protein. For example, by contacting a desired kinase with a monitor protein comprising the phosphorylation region to be phosphorylated by the enzyme in the presence of a test sample, property change of the monitor protein is detected. Any test samples can be used. Examples of the test sample include cellular extracts, expression products of gene libraries, synthetic low molecular compounds, synthetic peptides, modified peptides, natural compounds. A compound which stimulates or inhibits the property change is screened in comparison with the property change in the absence of the test sample. A compound which accelerates property change of the monitor protein is judged as a compound which stimulates kinase activity, and a compound which inhibits property change of the monitor protein is judged as a compound which inhibits kinase activity. This enables isolation of a compound which stimulates or inhibits phosphorylation activity of the kinase. Combinations of a kinase to be used for screening and a phosphorylation region are not particularly limited.

Examples of kinases used for screening in the present invention (and phosphorylation sequences thereof) include A kinase (CREB phosphorylation sequence of SEQ ID NO: 1), G kinase (RKRS^{*}RAE; histone), AMP activation protein kinase (HMRSAMS^{*}GLHLVKRR; acetyl CoAcarboxylase), calmodulin-dependent protein kinase II (PLRRTLS^{*}VAA; glycogen synthase), smooth muscle myosin light chain kinase (KKRAARATS^{*}NVFA; myosin light chain), phosphorylase kinase (KRKQIS^{*}VRGSL; phosphorylase), C kinase (VRKRT^{*}LRRL; EGF receptor), v-Ab1 (RRLIEDAEY^{*}AARG; RR60^{SRC}), EGF receptor protein kinase (RREELQDDY^{*}EDD; erythrocyte band III), but are not limited thereto (sequences and proteins in parentheses are substrate amino acid sequences and substrate proteins to be phosphorylated) (R. B. Rearson et al., "Specific studies on protein kinase and phosphatase using synthetic peptides"; D. G. Hardie Eds., supervised by Hidaka, H., MEDSi Biological Experiment Series, Protein Kinase and Phosphatase, pp225-228, 1995, Medical Science International). These kinases are known to phosphorylate proteins comprising an amino acid sequence other than the above, therefore, an amino acid sequence at these substrate sites can be appropriately selected and used.

In the case of screening *in vivo,* a vector carrying a nucleic acid which encodes a monitor protein can be used. In this system, for example, compounds which stimulate or inhibit signal transduction leading to expression and activation of the kinase, and compounds which stimulate or inhibit dephosphorylation of a monitor protein as well as compounds which directly stimulate or inhibit the activity of the target kinase (an agonist and antagonist of the kinase) can be screened. Whether a compound directly acts on a kinase or not can be confirmed by *in vitro* system using a purified kinase. In *in vivo* system, for example, in the case of using GFP as a variable property region as described in Examples, property change of a monitor protein can be detected without crushing cells. Moreover, property change of the monitor protein can be detected in real time in living cells. Effects of a test compound on phosphorylation can be examined in details in this manner.

By these screenings, for instance, an inhibitor specific to a particular kinase can be conveniently screened. Kinases have important functions in signal transduction in, for example, cellular proliferation, differentiation, and immunological responses. For example, an inhibitor to be isolated is expected to be applied as a drug for preventing or treating various diseases involved in signal transduction.

### Brief Description of the Drawings

Figure 1 shows the construction of pETIC vector.

Figure 2 shows. the purified recombinant protein separated by SDS-PAGE and stained with CBB.

Figure 3 shows the results of phosphorylation assay by A kinase.

Figure 4 shows the effect of the addition of A kinase on absorption wavelength for "A-Kinase Responsive Tracer (ART)" (derived from pETIC-ART).

Figure 5 shows the effect of addition of A kinase on absorption wavelength for "Kempart" (derived from pETIC-Kempart).

Figure 6 shows the effect of addition of A kinase on absorption wavelength for a negative control protein (derived from pETIC-1).

Figure 7 shows the construction of pCEP4.

Figure 8 shows the figure of ART in COS-7 living cells. (A) shows a BSGFP fluorescence image of ART expressed in a whole cells. (B) shows pseudo color images of BSGFP/RSGFP fluorescence intensity ratio in the cells in the same region as (A). The cells were treated with 5 µM dibutylyl-cAMP (db-cAMP) at hour 0. The bar indicates 30 µm.

Figure 9 shows the fluorescence intensity ratios of ART in COS-7 cells (mean±SEM, n=5) after the treatment with db-cAMP in the presence of H-89, which is a PKA inhibitor (the white bars), or the absence thereof (the black bars). Dibutylyl-cAMP (db-cAMP), which is a cAMP analogue, was added at hour 0. R indicates the florescence intensity ratio of cellular images detected in the region of about 10 µm X 10 µm square at the various times. Rmin indicates the minimum fluorescence intensity ratio of the same cells.

### Best Mode for Carrying Out the Invention

The present invention is illustrated in detail below with reference to Examples, but is not construed as being limited thereto.

In these Examples, phosphorylation was detected by constructing a monitor protein, A-kinase Responsive Tracer ("ART"), by using a phosphorylation sequence of CREB transcription factor (referred to CREB phosphorylation sequence hereafter) as a phosphorylation region and RSGFP and BSGFP of *Aequorea victoria* as a measurement protein pair. SEQ ID NO: 1 shows the CREB phosphorylation sequence.

A monitor protein "Kempart" was also constructed in the same manner using the phosphorylation sequence (SEQ ID NO: 2) of kemptide instead of the CREB phosphorylation sequence, and compared to the monitor protein containing the above CREB phosphorylation sequence.

### Examplel 1: Synthesis of a DNA fragment encoding a phosphorylation sequence

First, a DNA fragment encoding a phosphorylation sequence was synthesized to construct the above monitor protein. A DNA fragment encoding the CREB phosphorylation sequence (refer to the CREH-DNA fragment, hereafter) was amplified by PCR using the oligonucleotide LCR-1B (SEQ ID NO: 3) as a template, and PCR-1K (SEQ ID NO: 5) and PCR-1A (SEQ ID NO: 6) as primers for synthesis.

A DNA fragment encoding the phosphorylation sequence of kemptide (referred to as kemptide-DNA fragment hereinafter) used as a control was similarly amplified by PCR using the oligonucleotide LKe-1B (SEQ ID NO: 4) as a template, and a primer set of PKe-1K (SEQ ID NO: 7) and PKe-1A (SEQ ID NO: 8) as primers for synthesis. Detailed manipulation of PCR is as follows.

PCR reaction mixture was prepared in a microtube (for 0.2 ml). The composition of PCR reaction mixture was sterilized water (18.3 µl), 10 X EXTaq buffer (2.5 µl), dNTP mixture (2.0 µl), EXTaq polymerase (0.2 µl) (Takara), template oligonucleotides (20 nmol/ml) (1.0 µl), and primers (about 50 nmol/ml) (0.5 µl each).

A microtube containing the above reaction mixture was set on a DNA amplifier (GeneAmp PCR System 2400, Perkin Elmer Japan) and PCR was carried out. PCR was performed under the following conditions: 1 cycle of a denaturation process (94°C for 30 sec); 40 cycles of a series of a denaturation process (94°C for 50 sec), an annealing process (57°C for 1 min), and an extension process (72°C for 1 min); a final extension process (72°C for 7 min); and cooling process at 4°C.

After PCR, amplified fragments were confirmed by electrophoresis. Aportion of the reaction mixture (10 µl) was collected for electrophoresis and mixed with a buffer for electrophoresis. This mixture was separated on a 10% agarose gel and stained with ethidium bromide to confirm target fragments.

The target fragments were excised with a razor and such, and each of them was transferred to a microtube (for 1.5 ml) and recovered using Geneclean III kit (BIO 101). Each collected DNA was dissolved into Tris EDTA (TE) buffer (100 µl) and purified. Purification was conducted by the following procedure. First, a mixture of phenol, chloroform, and isoamyl alcohol (25: 24: 1) was added to the DNA solution and stirred. After standing it, supernatant was removed, and ethanol precipitation was conducted. Supernatant was removed, and precipitated pellet was dissolved in sterilized water.

### Example 2: Construction of a plasmid carrying a gene of a monitor protein

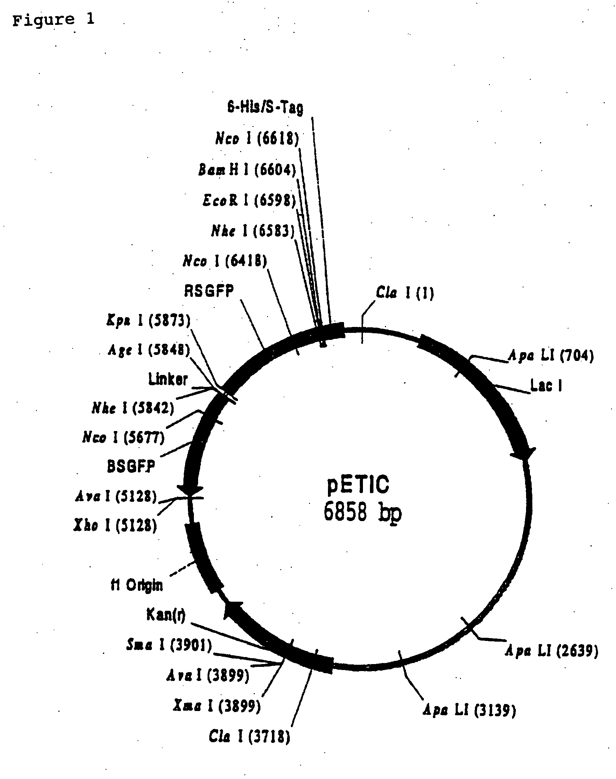

Each of the above DNA fragments was inserted into pETIC vector (J. Biol. Chem., 272: 13270-13274, 1997) shown in Fig. 1. This pETIC vector carries RSGFP gene and BSGFP gene as a measurement protein pair, and thus, a monitor protein gene in which "RSGFP gene - a phosphorylation sequence - BSGFP gene" was aligned is constructed by inserting the above DNA fragments between the RSGFP gene and BSGFP gene.

First, pETIC vector and above two DNA fragments were digested with restriction enzymes KpnI and AgeI according to the standard method. After digestion, each fragment and the linearized vector were separated on a 10% agarose gel by electrophoresis. After separation, each target fragment was collected with Geneclean III kit in the same manner as the above and purified. Each of these fragments was dissolved in TE buffer (10 µl).

Next, CREB-DNA fragment or kemptide-DNA fragment was ligated to the pETIC vector using the above DNA solution. Ligation was conducted with DNA Ligation Kit Ver. 2 (Takara).

The CREB fragment solution (5 µl) was mixed with the linearized pETIC vector solution (3 µl), and solution I (8 µl) of the above kit was added thereto to perform reaction at 16°C for 30 min. The kemptide fragment solution (5 µl) was mixed with the pETIC vector solution (3 µl) and solution I (8 µl) of the above kit to perform ligation reaction in the same manner. The ligation sample only with TE buffer instead of the insert was also prepared.

To collect a target plasmid from the above ligation reaction solution, the above ligation reaction solution (8 µl) was mixed with JM109 competent cells (100 µl), and transformation was conducted according to the standard method. After transformation, the JM109 cells were spread on an agar medium containing kanamycin and cultured at 37°C overnight. After incubation, kanamycin-resistant colonies grown were taken, and the plasmids in the cells were analyzed. By this analysis, the cells containing plasmids in which the target fragments were inserted into pETIC vector were selected, and the plasmids were prepared from these cells. Among the plasmids prepared in this manner, the plasmid into which the CREB fragment was inserted and the plasmid into which the kemptide fragment was inserted were designated "pETIC-ART" and "pETIC-Kempart," respectively.

### Example 3: Production of a monitor protein

Monitor proteins comprising the phosphorylation sequence derived from either CREB or kemptide were produced using the above "pETIC-ART" or "pETIC-Kempart," respectively. The pETIC vector, a vector for these plasmids, was prepared based on pET30a vector (Novagen), and has the functions of pET30a. Specifically, in the pETIC vector, T7 promoter is provided upstream of the insert region, and expression of a target protein can be induced with IPTG in *E. coli* containing T7 RNA polymerase gene downstream of lacUV5 promoter, such as BL21(DE3). The recombinant proteins thus expressed can be easily collected with Ni agarose because histidine tag (His-tag) is added to the proteins. The monitor proteins were produced using "pETIC-ART" and "pETIC-Kempart" by the following procedure based on the above principle.

BL21(DE3) competent cells were transformed with the above "pETIC-ART" or "pETIC-Kempart" using the same method as mentioned above. After transformation, colonies formed on the kanamycin-containing agar medium were taken, inoculated into 3 ml of LB medium (kanamycin 50 µg/ml), and incubated at 37°C overnight to prepare the preculture medium. This preculture medium (2 ml) was transferred into a 2-L flask containing 500 ml of the same LB medium, and cultured at 37°C until absorbance at 600 nm reached 0.6. IPTG was added thereto (concentration: 1 mM) when absorbance at 600 nm was 0.6, and culturing was further performed at 23°C overnight.

The cultured medium was centrifuged (5,000 rpm, 10 min, 4°C), and cells were harvested. The collected cells were suspended in 10 ml of a buffer (50 mM Tris-HCl buffer, pH 7.5, 150 mM NaCl, 2 mM benzamidine, 1 mM PMSF). The cells in the resulting suspension were disrupted by sonication on ice for five times under the condition of a 30-second pulse. The extract was mixed with NP-40 and imidazole with the final concentration of 0.1% and 20 mM, respectively, stirred with a stirrer at 4°C, and centrifuged (10,000 rpm, 10 min) at 4°C to collect supernatant.

The collected supernatant was mixed with 500 µl of Ni-NTA-agarose (Qiagen) and stirred with a stirrer at 4°C for 60 min. After stirred, the above Ni-NTA-agarose was washed with 2 ml of a binding buffer (50 mM Tris-HCl, pH 7.5, 150 mM NaCl, 2 mM benzamidine, 1 mM PMSF, 0.1% NP-40, 20 mM imidazole) four times.

After washed, the agarose was mixed with 1 ml of an elution buffer (50 mM Tris-HCl at pH 7.5, 150 mM NaCl, 2 mM benzamidine, 1 mM PMSF, 0.1% NP-40, 200 mM imidazole), stirred at 4°C with a stirrer for 30 min, and centrifuged to collect supernatant. This series of elution procedures was repeated twice. The collected eluate was mixed with glycerol at the final concentration of 20%, and stored at -80°C.

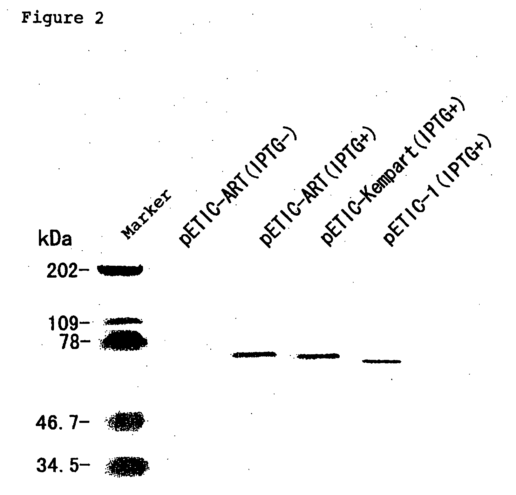

Ten microliters of the eluate collected in the above manner was subjected to the 10% acrylamide-gel electrophoresis to confirm a monitor protein. After electrophoresis, the gel was stained with Coomassie Brilliant Blue (CBB). The results were shown in Fig. 2. In Fig. 2, pETIC-1 (Romeser, V. A. et al, J. Biol. Chem., 272: 13270-13274) was the pETIC vector into which a sequence encoding the Ca²⁺ calmodulin binding site was inserted, and used as a positive control in the preparation of the recombinant protein (RSGFP - the calmodulin binding site - BSGFP). "IPTG-" and "IPTG+" indicate the absence and the presence, respectively, of induction with IPTG during the incubation.

As shown in Fig. 2, in the case of expression induction with IPTG, the protein shown as a single band was detected in each of "pETIC-ART" and "pETIC-Kempart." The sizes of these proteins were similar to those of the proteins expressed by pETIC-1, which is a positive control. Therefore, the proteins expressed here were expected to be the target monitor proteins, namely RSGFP - CREB phosphorylation sequence - BSGFP and RSGFP - kemptide phosphorylation sequence - BSGFP.

### Example 4: Measurement of phosphorylation using γ-³²P

It was examined whether the phosphorylation sequences in the above two monitor proteins would be phosphorylated or not in the *in vitro* system. This examination was conducted using A kinase as a kinase and [γ-³²P]ATP as a substrate according to the following procedure.

To prepare the reaction solution, 10 X kinase buffer (250 mM Hepes, pH 7.5, 100 mM MgCl₂, 10 mM DTT) (5 µl), A kinase (1 µl), [γ-³²P]ATP (0.5 µl), and sterilized water (43 µl) were mixed. To this mixture, each eluate (0.5 µl) containing each monitor protein was added, and incubated at 30°C for 90 min to carry out phosphorylation reaction.

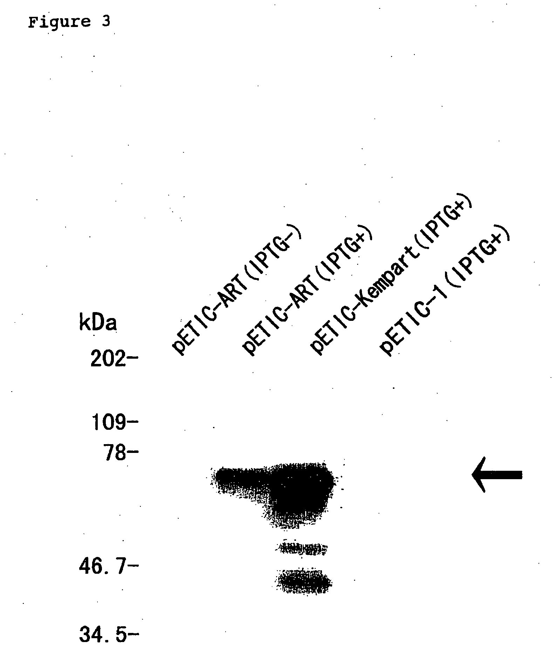

After the reaction, whether each monitor protein was phosphorylated or not was examined by electrophoresis. As a pre-treatment for electrophoresis, the reaction solution after the reaction (50 µl) was mixed with 2 X sample buffer (50 µl) and boiled for 3 min. After boiling, the solution (20 µl) was subjected to electrophoresis with the 10% acrylamide gel. After electrophoresis, the gel was exposed to films to detect signals of γ-³²P (Fig. 3).

As shown in Fig. 3, signals were detected both for the monitor protein derived from "pETIC-ART" and for the monitor protein derived from "pETIC-Kempart." Specificity of these signals were obvious by the fact that signals were not detected for pETIC-1-derived protein which did not contain any phosphorylation sequence. The respective positions of the signals detected for "pETIC-ART" and "pETIC-Kempart" corresponded to the size of the monitor proteins expressed by the plasmids shown in Fig. 2. These results confirmed that the two monitor proteins produced in Example 3 maintained the phosphorylation sequences and that the sequences were phosphorylated.

### Example 5: Measurement of phosphorylation by fluorescence change

One end of the phosphorylation sequence in the above two monitor proteins was ligated to RSGFP and the other to BSGFP. Fluorescence change due to interference of RSGFP and BSGFP at the each side by phosphorylation of the phosphorylation sequence in these two monitor proteins was examined.

As a reaction solution, 10 X kinase buffer (20 µl), ATP (100 mM) (2 µl), and sterilized water (168 µl) were mixed and the above monitor proteins (5 µg/µl) (10 µl) were added to this mixture. Moreover, to this reaction solution, 0, 1, 2, or 4 µl of A kinase were added.

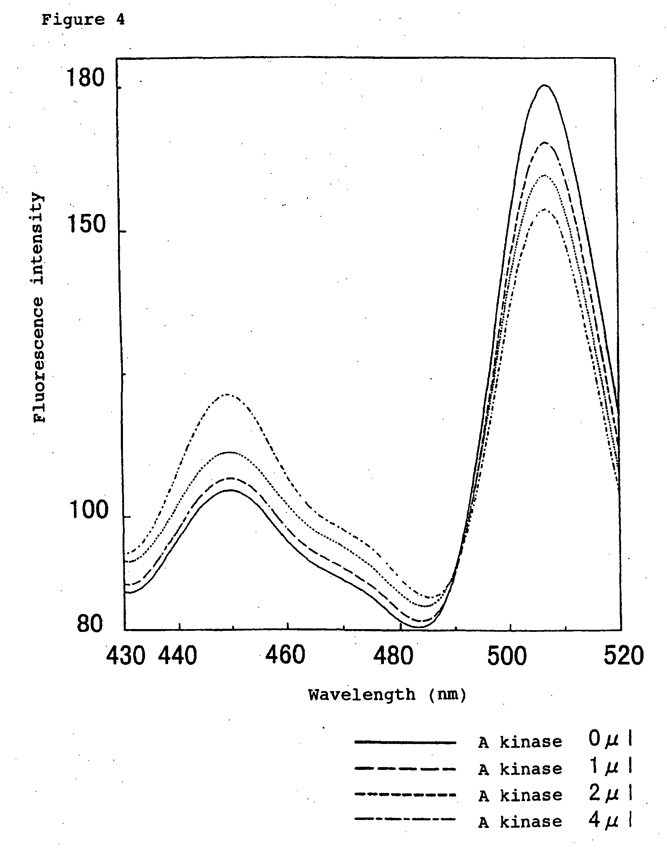

The prepared reaction solution was incubated at 30°C for 30 min. Changes in the range of wavelength absorbed at the range of wavelength between 430 nm and 520 nm were measured in the incubated reaction solution with a fluorescence spectrophotometer (Nippon Bunnko). Figures 4, 5, and 6 show the results for fluorescence changes of "ART" (derived from pETIC-ART), "Kempart" (derived from pETIC-Kempart), and pETIC-1 derived recombinant protein, which is a negative control, respectively. In the above figures, the difference in the amounts of A kinase added was expressed with the different lines.

As shown in Fig. 4, in "ART," a difference in the absorbance patterns was observed between the case that A kinase was not added and the case that A kinase was added. When A kinase was not added, the stronger absorbance was observed at 500 nm. As the amount of added A kinase increased, the absorbance at 450 nm was enhanced. This indicates that conformation change was generated by phosphorylation of the CREB phosphorylation sequence, and thereby RSGFP and BSGFP at the either end could interfere each other, emitting fluorescence.

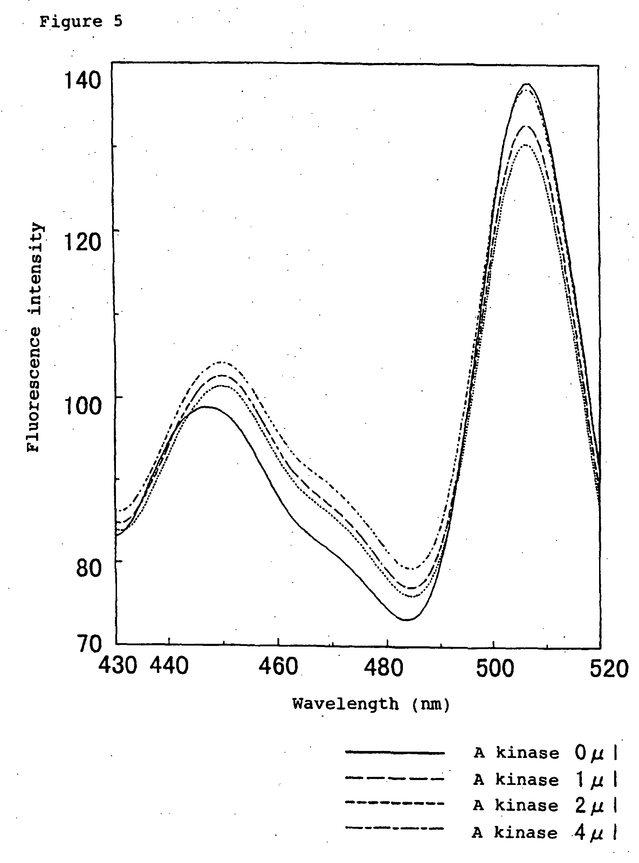

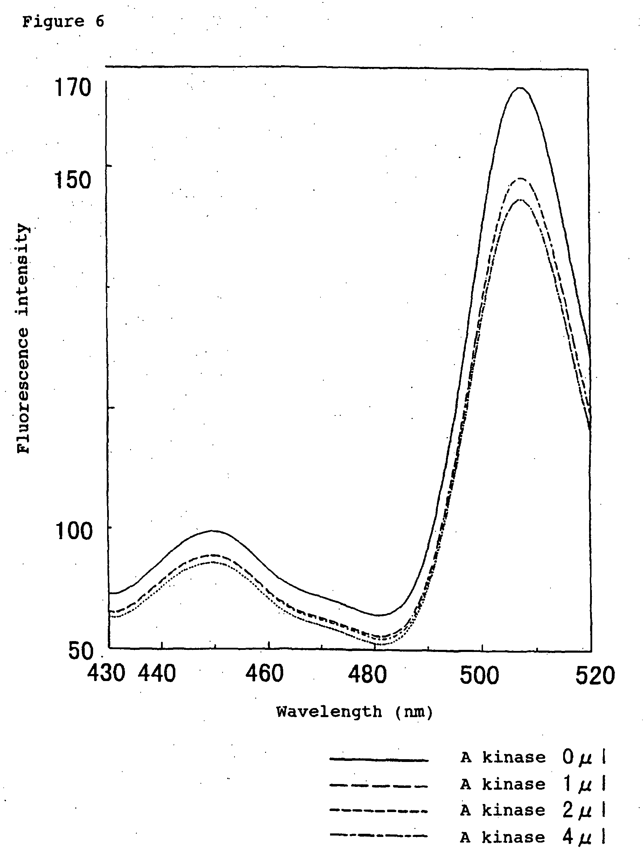

In contrast, in "Kempart" (Fig. 5), although phosphorylation was confirmed in the phosphorylation measurement using [γ-³²P]ATP in Example 3 above, no fluorescence change was observed in the above-described wavelength range as in the negative control (Fig. 6). From these results, it was expected that the phosphorylation sequence of kemptide was phosphorylated, but the phosphorylation was not accompanied with conformational change.

Thus, it has been revealed that the above "ART" can be used for, for instance, detecting phosphorylation reaction using the above-described fluorescence change as an indicator. *In vivo* system can be constructed by introducing pETIC-ART, which expresses this "ART", into cells. Moreover, the above "ART" can be used not only for detecting phosphorylation reaction, but also for screening kinases and screening accelerators and inhibitors of phosphorylation activity.

On the other hand, it was shown that there was a difference between CREB phosphorylation sequence and kemptide phosphorylation sequence in terms of the presence and absence of conformational change at the time of phosphorylation. This finding indicates that RSGFP and BSGFP can be used for analyzing conformational change in phosphorylation sequences upon phosphorylation. Specifically, it was shown that conformational change at the time of phosphorylation can be observed by inserting any phosphorylation sequence between RSGFP and BSGFP and by performing both phosphorylation measurements using [γ-³²P]ATP and those based on fluorescence change.

### Example 6: In vivo measurement of phosphorylation

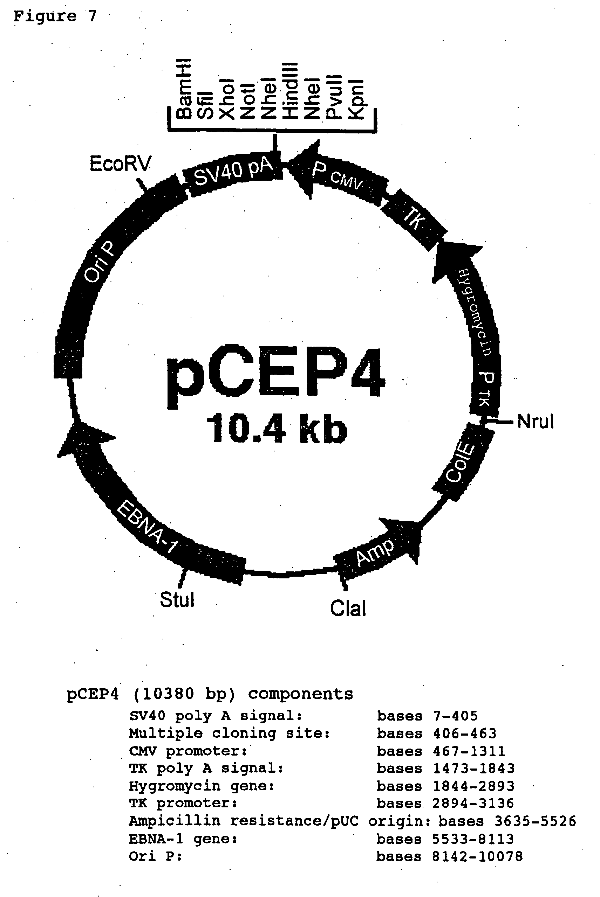

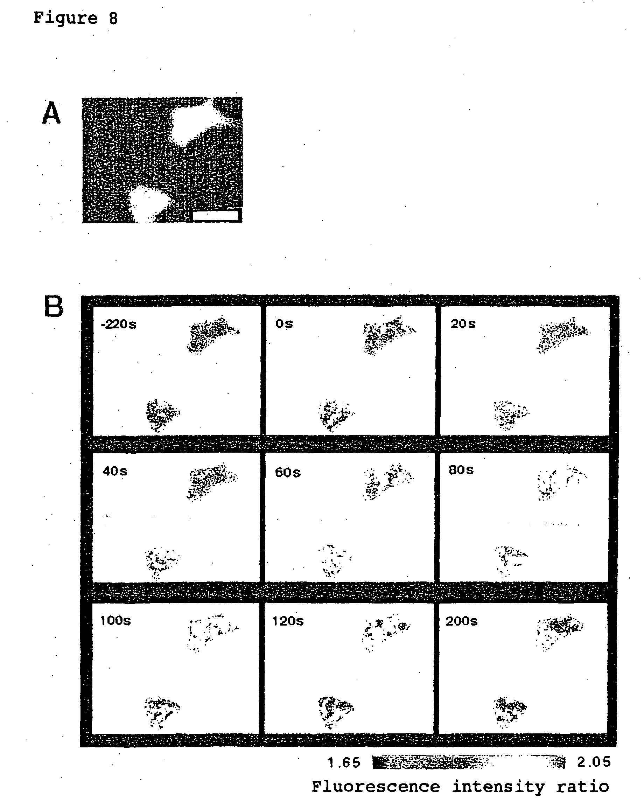

1) Structure and construction of pCEP4-ART
   The pETIC-ART plasmid and the mammalian expression vector pCEP4 (Invitrogen) plasmid vector (Fig. 7) were digested with the restriction enzymes HindIII and XhoI and electrophoresed on a 10% agarose gel to collect the target bands with Geneclean III kit. The collected DNA was dissolved in 10 µl of TE, and 8 µl of the DNA solution was mixed with 8 µl of solution I of DNA Ligation Kit Ver. 2 (Takara) to perform ligation reaction at 16°C for 30 min. Eight microliters of the solution were mixed with 100 µl of JM109 *E*. *coli* competent cells, and the cells were transformed. The cells were spread on an ampicillin plate and cultured at 37°C overnight. Colonies were then taken and cultured in 3 ml of LB medium (ampicillin 50 µg/ml) at 37°C overnight. Two milliliters of the overnight cultured solution were added into 500 ml of LB medium (ampicillin 50 µg/ml) in a 2-liter flask, and cultured at 37°C overnight. *E. coli* was collected and the plasmid DNA (pCEP4-ART) was collected with Qiagen Tip (Qiagen).
2) Introduction of plasmid DNA into COS-7 cells
   One microgram of pCEP4-ART plasmid DNA was added to 100 µl of serum-free DMEM medium, and 4 µl of lipofectAMINE regent (GIBCO BRL) were added to another 100 µl of serum-free DMEM medium. These two DMEM media were mixed with each other and stood for 30 to 40 min at room temperature. Then, the medium in a glass-bottomed culture dish (MatTek) in which COS-7 cells had been passage-cultured from the day prior to DNA introduction was replaced with 800 µl of serum-free DMEM medium, and the mixture of plasmid DNA and lipofectAMINE reagent was added thereto to culture for 3 hours. The medium was replaced with 1 ml of DMEM medium containing 10% bovine serum, and culturing was performed for 2 days.
3) Imaging of phosphorylation
   COS-7 cells transfected with pCEP-4 ART were spread on a cover slip (a diameter of 14 mm) of a glass-bottomed culture dish (MatTek). The cells were incubated at 30°C for 48 hours and rinsed twice with Ringer's buffer (150 mM NaCl, 4 mM KCl, 2 mM CaCl₂, 1 mM MgCl₂, 5 mM Hepes, pH 7.4, and 5.6 mM glucose), and kept in the buffer in the dark at room temperature. Fluorescence intensity was measured with excitation light at 380 nm by detecting with 440 ± 20-nm and 520 ± 20-nm band pass filters using a 100 X oil immersion objective lens (OLYMPUS UV-Fluor), a Quantix CCD camera (Roper Scientific), and an IPLab Spectrum image processor (Scanalytics). Exposure duration for excitation light at 380 nm was 0.2 sec, and exposure interval was 20 sec. An image passed through the 440 ± 20 nm filter was detected, and after 5 seconds, an image passed through 520 ± 20 nm filter was detected. Cells were induced with dibutylyl-cAMP (db-cAMP), a cAMP analogue, by adding 500 µl of Ringer's buffer containing 10 µM db-cAMP to a culture dish containing 500 µl of Ringer's buffer.
4) Results
   Observation for intracellular distribution of "ART" revealed that "ART" was dispersed in the COS-7 cells (Fig. 8A). The concentration of intracellular cyclic-adenosine monophosphate (cAMP) was increased with db-cAMP, an analogue of cAMP, to activate A kinase, and fluorescence change in the cells was measured in real time with a cooled CCD camera (Fig. 8B). db-cAMP was added at the time of 0 sec. Fluorescence intensity ratios in the cellular image indicate that values of fold induction (fluorescence intensity ratio) increased depending on the time after the addition of db-cAMP. The increase in fold induction was transmitted from the side of the cell membrane to the nucleus, coinciding with the phenomenon that an active form A kinase moves from the cytoplasm to the nucleus after the increase of cytoplasmic cAMP. The average fold induction increased with time after db-cAMP stimulation. This indicates that "ART" can be used as phosphorylation monitor system in a living cell.

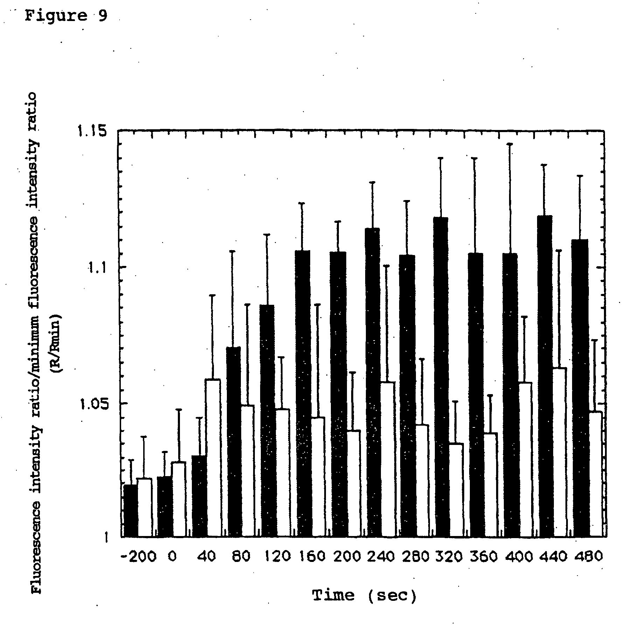

To examine whether "ART" is actually phosphorylated in COS-7 cells depending on A kinase, intracellular A kinase was inhibited with H-89 which is a selective inhibitor of A kinase, to perform the experiment for db-cAMP stimulation in the same manner as Fig. 8. The same experiment as described above was conducted except that the cells were pre-treated about for 2 hours with DMEM containing 10 µM H-89 and that, then, the medium was replaced with Ringer's buffer containing 10 µM H-89. Fluorescence change of "ART" in the cells was measured with a cooled CCD camera in real time (Fig. 9). Comparison of fluorescence intensity ratios in the region of 10 µm X 10 µm range indicates that fluorescence intensity ratio was low in the presence of H-89, showing that inhibition of A kinase activity can be observed in a living cell in real time.

As shown above, using the monitor proteins of the present invention, the system for monitoring phosphorylation in vivo was constructed. Using this system, phosphorylation can be easily monitored under the environment more similar to physiological conditions. Moreover, it was shown that effects of inhibitors on kinase can be easily monitored using this system. Therefore, this system is useful for screening a compound which stimulates or inhibits phosphorylation.

### Industrial Applicability

In the present invention, an analysis system in which no radioactive isotopes are used and that is applicable to in vivo measurement has been developed for protein phosphorylation'. The analysis system of the present invention can be used not only for the detection of phosphorylation reaction, but also for screening a kinase and screening a compound which stimulates or inhibits phosphorylation.

## Claims

1. A monitor protein for measuring protein phosphorylation, the monitor protein comprising (a) a phosphorylation region comprising an amino acid residue or an amino acid sequence to be phosphorylated, and (b) a variable property region showing a property change attributed to a conformational change of a protein comprising at least the phosphorylation region, which conformational change is caused by phosphorylation of the amino acid residue or the amino acid sequence.

2. The monitor protein of claim 1, wherein the variable property region is a protein that emits fluorescence.

3. The monitor protein of claim 1 or 2, wherein the variable property region is bound to each of both ends of the phosphorylation region.

4. The monitor protein of claim 3, wherein the variable property region comprises RSGFP and BSGFP which are comprised in green fluorescent protein (GFP) of *Aequorea victoria*.

5. The monitor protein of any one of claims 1 to 4, wherein the phosphorylation region comprises the amino acid sequence of SEQ ID NO: 1.

6. A nucleic acid encoding the monitor protein of any one of claims 1 to 5.

7. An expression vector carrying the nucleic acid of claim 6.

8. A method for measuring phosphorylation ability in a cell by introducing the monitor protein of any one of claims 1 to 5, the nucleic acid of claim 6, or the expression vector of claim 7 into the cell.

9. A method for measuring phosphorylation ability of a test protein, the method comprising reacting the test protein with the monitor protein of any one of claims 1 to 5, and measuring a property change of the monitor protein.

10. A method for screening a kinase, the method comprising:
(a) reacting a test protein with the monitor protein of any one of claims 1 to 5,
(b) measuring the property change of the monitor protein, and
(c) selecting the test protein which alters the property of the monitor protein.

11. A method for screening a compound which stimulates or inhibits phosphorylation, the method comprising:
(a) contacting, in the presence of a test sample, a kinase with the monitor protein of any one of claims 1 to 5, the monitor protein comprising a phosphorylation region to be phosphorylated by the kinase,
(b) measuring the property change of the monitor protein, and
(c) selecting a compound which stimulates or inhibits the property change in comparison with the property change in the absence of the test sample.

12. A method for screening a compound which stimulates or inhibits phosphorylation, the method comprising:
(a) preparing a cell into which the expression vector of claim 7 is introduced,
(b) measuring, in the presence of a test sample, the property change of a monitor protein expressed in the cell, and
(c) selecting a compound which stimulates or inhibits the property change in comparison with the property change in the absence of the test sample.
